# EUROPEAN PATENT SPECIFICATION

(11) **EP 4 563 290 B1**
(45) Date of publication and mention of the grant of the patent: **18.11.2026**
(21) Application number: 22956451.3
(22) Date of filing: 23.08.2022
(51) Int. Cl.: B25J 3/00, A61B 34/37, A61B 34/00

(54) **SURGICAL ROBOT AND CONTROL UNIT FOR SURGICAL ROBOT**
CHIRURGISCHER ROBOTER UND STEUEREINHEIT FÜR CHIRURGISCHEN ROBOTER
ROBOT CHIRURGICAL ET UNITÉ DE COMMANDE POUR ROBOT CHIRURGICAL

(43) Date of publication of application: 04.06.2025
(73) Proprietor: RIVERFIELD Inc., 1070052 Tokyo (JP)
(72) Inventor: TADANO, Kotaro, Tokyo 107-0052 (JP); KANAZAWA, Masao, Tokyo 107-0052 (JP)
(74) Representative: Adares PartGmbB
(86) International application number: PCT/JP2022/031775
(87) International publication number: WO 2024/042623

(56) References cited:
- JP-A- 2009 285 099
- JP-A- H0 871 072
- JP-A- H1 029 177
- US-A1- 2003 004 610
- US-A1- 2005 200 324
- US-A1- 2013 312 563
- US-A1- 2015 018 841
- US-A1- 2017 050 310
- US-A1- 2020 237 456
- US-B1- 6 424 885
- CHAWDA VINAY ET AL: "Position Synchronization in Bilateral Teleoperation Under Time-Varying Communication Delays", IEEE/ASME TRANSACTIONS ON MECHATRONICS IEEE SERVICE CENTER, PISCATAWAY, NJ., US, vol. 20, no. 1, 1 February 2015 (2015-02-01), pages 245 - 253, XP011560402, ISSN: 1083-4435, [retrieved on 20141003], DOI: 10.1109/TMECH.2014.2317946

## Description

### TECHNICAL FIELD

The present disclosure relates to a master-slave type surgical robot.

### BACKGROUND ART

The master-slave type surgical robot receives an operation from a medical worker such as a doctor (hereinafter, referred to as "user") in a master, and uses a surgical tool that is an instrument for surgery, such as a pair of forceps or a scalpel, as a slave. The slave is controlled according to the operation received in the master, and surgery is performed on a patient (hereinafter, referred to as "subject"). A technique is known in which, in such a surgical robot, a force received by the surgical tool during surgery is returned from the slave to the master through a bilateral control, thereby providing force feedback to the user (e.g., Patent Document 1).

In the master and the slave of the surgical robot that performs such force feedback, a proportional control is performed. In order to improve the sensitivity of the force feedback, it is conceivable that a gain of the proportional control of the master is increased.

Further, Patent Document 2 describes a master-slave system having a master robot and a slave robot and being subjected to bilateral control. The system comprises a master actuator for generating a master driving force to control the master robot in terms of joint displacement, a slave actuator for generating a slave driving force to control the slave robot in terms of driving force; several master sensors, and several slave sensors. The master actuator is adapted to generate a master driving force on the basis of a master target displacement and a master displacement, whereas the slave actuator is adapted to generate a slave driving force on the basis of a slave target effective driving force and a slave effective driving force.

Patent Document 3 describes a surgical robot system, comprising a slave device provided with surgical tools, and a master device configured to remotely control motion of the surgical tools. The master device includes a handle configured to control the motion of the surgical tools, a master external force estimation unit configured to estimate an external force applied to the handle, a force compensator configured to generate a first force control signal that cancels out the estimated external force, and a master controller configured to control moving and rotating of respective joints of the handle in such a way that the external force applied to the handle is cancelled out using the generated first force control signal.

Further, Patent Document 4 describes a system comprising a master manipulator and a slave manipulator connected to each other in a bilaterally controllable manner. The master operator is operated primarily under electrically-driven speed control and the slave manipulator is operated primarily under pneumatically-driven force control. An external force acting on the slave manipulator is fed back into the master manipulator. The system comprises an external force estimation section, a master manipulator drive control unit, and a slave manipulator drive control unit.

Patent Document 5 describes a medical device, and a slave manipulator adapted to manipulate the medical device, an input device operatively coupled to the slave manipulator, and a processor programmed to dynamically adjust a characteristic of the slave manipulator. Other relevant prior art can be found in the patent publications US 2003/004610 A1 and US 2005/200324 A1, and also in the scientific publication titled "Position Synchronization in Bilateral Teleoperation Under Time-Varying Communication Delays" by Chawda et al.

### PRIOR ART DOCUMENT

### PATENT DOCUMENT

Patent Document 1: Japanese Patent No. 7049069
Patent Document 2: US 2017/050310 A1
Patent Document 3: US 2015/018841 A1
Patent Document 4: US 2013/312563 A1
Patent Document 5: US 2020/237456 A1

### SUMMARY OF THE INVENTION

### PROBLEMS TO BE SOLVED BY THE INVENTION

However, for example, if a wire for transmitting a driving force to the surgical tool is slack, and/or there is a gap between the surgical tool and a transmission mechanism of the driving force, and/or there is a delay in the control, an excessive increase in the gain of the proportional control of the master may cause difficulty in controlling the surgical tool. Therefore, there is a limit to increasing the gain of the proportional control.

In one aspect of the present disclosure, it is desirable to improve the sensitivity of the force feedback.

### MEANS FOR SOLVING THE PROBLEMS

The problem is solved by a controller according to claim 1 and a surgical robot according to claim 2. One aspect of the present disclosure is a surgical robot including a master, a master controller, a slave, a slave controller, and a slave detector. The master is configured to receive an operation from a user and to apply a reaction force to the user performing the operation. The master controller is configured to control the master and to cause the reaction force to be generated. The slave is configured to actuate a surgical tool. The slave controller is configured to control the slave based on a target state, the target state being a state of the surgical tool based on the operation received by the master, the slave controller being configured to cause the surgical tool to be actuated. The slave detector is configured to detect a surgical tool state that is a state of the surgical tool. The slave controller is configured to calculate a slave control value based on the surgical tool state and the target state, the slave control value being for controlling the surgical tool, the slave controller being configured to control the slave based on the slave control value. The master controller is configured to calculate a master control value based on the surgical tool state, the target state, and the slave control value obtained from the slave controller, the master control value being for controlling the reaction force, the master controller being configured to control the master based on the master control value.

In the above configuration, the master control value is calculated by taking the slave control value into consideration, and the reaction force to the user is controlled by the master control value. This allows the changes in the slave control value to be reflected more quickly in the master control value, and as a result, the sensitivity of the force feedback improves.

In one aspect of the present disclosure, the slave controller may be configured to calculate the slave control value by performing an integration based on the surgical tool state and the target state.

In the above configuration, a disturbance (in other words, a steady-state error) in the slave controller can be reduced. Therefore, it is possible to calculate the slave control value more accurately.

In one aspect of the present disclosure, the slave controller may be configured to calculate the slave control value by a proportional control based on a difference between the surgical tool state and the target state. The master controller may be configured to calculate the master control value by a proportional control based on the difference between the surgical tool state and the target state. A gain of the proportional control in the master controller may be greater than a gain of the proportional control in the slave controller.

In the above configuration, the sensitivity of the force feedback improves.

In one aspect of the present disclosure, the master controller may be configured to calculate the master control value by a proportional control based on a difference between the surgical tool state and the target state. The surgical robot may further include a display and an input section. The display is configured to display a gain of the proportional control. The input section is configured to receive an operation to change the gain of the proportional control.

In the above configuration, the sensitivity of the force feedback can be adjusted.

In one aspect of the present disclosure, the slave controller may be configured to calculate the slave control value by a proportional control based on a difference between the surgical tool state and the target state. The surgical robot may further include a display and an input section. The display is configured to display a gain of the proportional control. The input section is configured to receive an operation to change the gain of the proportional control.

The above configuration allows customization for better control of the surgical tool.

In one aspect of the present disclosure, the slave controller may be configured to calculate the slave control value by further performing an integral control based on the difference between the surgical tool state and the target state. The display may be configured to further display a gain of the integral control. The input section may be configured to further receive an operation to change the gain of the integral control.

The above configuration allows customization for better control of the surgical tool.

One aspect of the present disclosure is a controller for a surgical robot, the controller including a master controller and a slave controller. The master controller is configured to control a master, the master being configured to receive an operation from a user and to apply a reaction force to the user performing the operation, the master controller being configured to cause the reaction force to be generated. The slave controller is configured to control a slave based on a target state, the slave being configured to actuate a surgical tool, the target state being a state of the surgical tool based on the operation received by the master, the slave controller being configured to cause the surgical tool to be actuated. The slave controller is also configured to calculate a slave control value based on a surgical tool state and the target state, the slave control value being for controlling the surgical tool, the surgical tool state being a state of the surgical tool detected by a slave detector, the slave controller being configured to control the slave based on the slave control value. The master controller is configured to calculate a master control value based on the surgical tool state, the target state, and the slave control value obtained from the slave controller, the master control value being for controlling the reaction force, the master controller being configured to control the master based on the master control value.

In the above configuration, the master control value is calculated by taking the slave control value into consideration, and the reaction force to the user is controlled by the master control value. This allows the changes in the slave control value to be reflected more quickly in the master control value and, as a result, the sensitivity of the force feedback improves.

### BRIEF DESCRIPTION OF THE DRAWINGS

FIG. 1 is a diagram illustrating a surgical robot.
FIG. 2 is a block diagram of the surgical robot.
FIG. 3 is a diagram illustrating a master-slave modeled in a one-degree-of-freedom system.
FIG. 4 is a block diagram of an asymmetric bilateral control.
FIG. 5 is a diagram illustrating a gain setting screen.

### EXPLANATION OF REFERENCE NUMERALS

1...surgical robot, 2...robot arm, 20...connector, 21...arm, 22...articulation, 23-25...first to third drivers, 26...sensor, 3...controller, 30...master controller, 31...slave controller, 32... gain adjustor, 33...storage device, 4...operation unit, 40...operation device, 41...position operation section, 41A...movable part, 41B...driver, 42...grasp operation section, 42A...movable part, 42B...driver, 43...display, 44...input section, 5...surgical tool, 50...grasper, 50A...sensor, 51...opening/closing mechanism, 52... wrist, 52A...sensor, 53... shaft, 54...adapter, 6...trocar, 7...setting screen, 70-75...display area, 76...cursor

### MODE FOR CARRYING OUT THE INVENTION

Hereinafter, embodiments of the present disclosure are described with reference to the drawings. It should be noted that the embodiments of the present disclosure are not limited in any way to the following embodiments, and may take various forms as long as they fall within the technical scope of the present disclosure.

### [1. Overview]

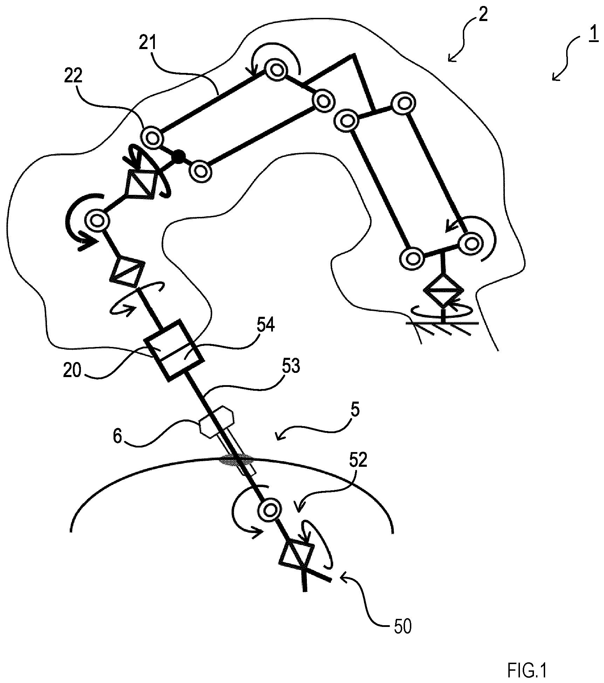

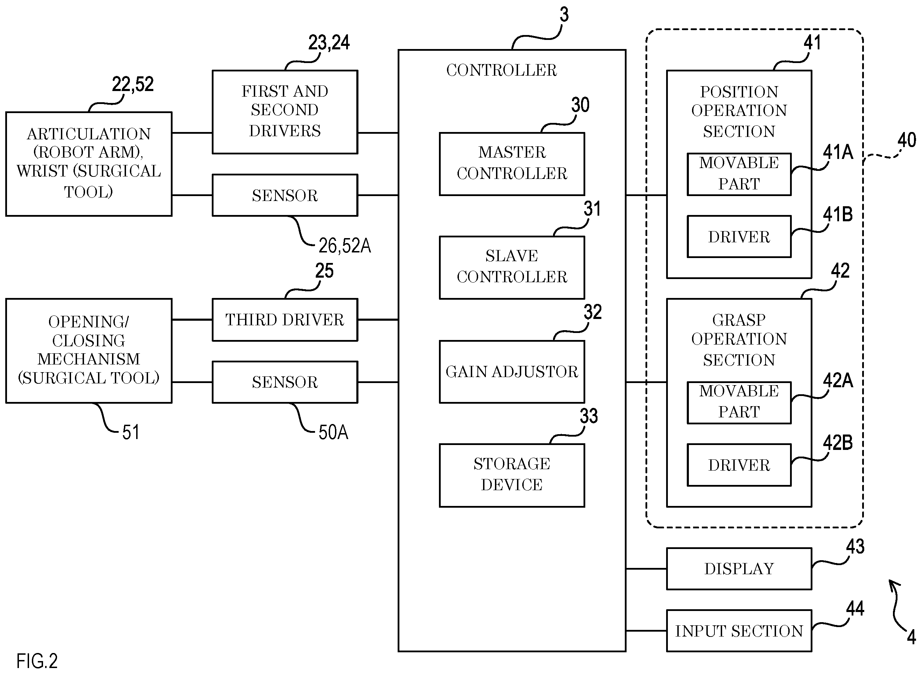

A surgical robot 1 of this embodiment includes two robot arms 2 having the same configuration, a controller (control unit) 3, and an operation unit 4 (see FIGS. 1 and 2). Each of the robot arms 2 is equipped with a surgical tool 5. It should be noted that the number of robot arms 2 may be one, or three or more.

The surgical robot 1 is configured as a master-slave type robot system. The surgical robot 1 actuates the robot arm 2 and the surgical tool 5 (i.e., a slave) in response to the user operating an operation section of the operation unit 4 (i.e., a master) to thereby perform surgery on a subject. The surgical robot 1 is also configured to perform force feedback. As described in detail below, during surgery, the surgical robot 1 is configured to allow the user to perceive a force received by the surgical tool actuated in accordance with the user's operation through the operation section of the operation unit 4.

### [2. Surgical Tool]

The surgical tool 5 is configured such that its tip is inserted into the subject's body through a trocar 6 punctured in the subject's abdomen or the like, and a part provided at the tip is actuated within the body, thereby performing a surgical treatment (see FIG. 1). In this embodiment, as an example, a pair of forceps is used as the surgical tool 5. However, it is not limited thereto; for example, a surgical tool other than the forceps, such as a scalpel, may also be used.

The surgical tool 5 includes a grasper 50, an opening/closing mechanism 51, a wrist 52, a shaft 53, and an adapter 54 (see FIGS. 1 and 2).

The shaft 53 is an elongated cylindrical portion and provided with an adapter 54 at the end opposite the tip.

The grasper 50 is provided at the tip of the shaft 53 and inserted through the trocar 6 into the subject's body to perform a treatment, such as grasping the subject's internal tissues. The grasper 50 includes first and second portions and the opening/closing mechanism 51, and the opening/closing mechanism 51 is configured to move the first and second portions closer together and farther apart (hereinafter, also referred to as "opening/closing movement"). The first and second portions move closer together to thereby grasp an object, and the grasped object is released by the first and second portions moving apart.

The grasper 50 also includes a sensor 50A for detecting the state of the opening/closing movement (hereinafter, referred to as "opening/closing state") (see FIG. 2). Specifically, for example, the sensor 50A may detect a distance between the first portion and the second portion, and/or detect an angle between the first portion and the second portion as the opening/closing state.

The wrist 52 is provided near the grasper 50 in the shaft 53, and is rotatable at one or more locations, and changes the orientation (i.e., posture) of the grasper 50 by rotating at the location. The wrist 52 also includes a sensor 52A for detecting the orientation of the grasper 50.

The sensors 50A, 52A output sensor signals indicating detection results to the controller 3.

The cylindrical shaft 53 contains a plurality of wires that transmits a driving force to the grasper 50 and the wrist 52 to actuate these parts.

### [3. Robot Arm]

The robot arm 2 is configured to hold the surgical tool 5, which is mounted on the tip, at a specified position and to change the posture of the robot arm 2 in response to the user's operation to thereby adjust the position and orientation (i.e., posture) of the surgical tool 5 (see FIG. 1).

The tip of the robot arm 2 is provided with a connector 20 that can be attached to and detached from an adapter 54 of the surgical tool 5, and the surgical tool 5 is mounted through the connector 20. Various types of forceps and surgical tools other than forceps can be mounted on the connector 20 of the robot arm 2.

The robot arm 2 is configured as a link mechanism, and includes two or more arms 21 and two or more articulations 22 (i.e., joints) that are provided at the ends of the arms 21 and that rotate each arm 21. Each arm 21 is coupled to another arm 21 through the articulation 22.

The robot arm 2 also includes first to third drivers 23-25 (see FIG. 2). The first to third drivers 23-25 may include, for example, at least one pneumatic actuator, at least one motor, or the like.

The first driver 23 is configured to drive each articulation 22 in response to the user's operation, and the driving force generated by the first driver 23 is transmitted to each articulation 22 through the transmission mechanism including the plurality of wires and a plurality of pulleys. The first driver 23 drives each articulation 22 to thereby displace each arm 21 and change the posture of the robot arm 2, thereby changing the orientation and position of the surgical tool 5.

The second driver 24 is configured to drive the wrist 52 of the surgical tool 5 in response to the user's operation, and the driving force generated by the second driver 24 is transmitted to the wrist 52 through the transmission mechanism. The second driver 24 drives the wrist 52, thereby changing the orientation of the grasper 50.

The third driver 25 is configured to drive the opening/closing mechanism 51 of the surgical tool 5 in response to the user's operation, and the driving force generated by the third driver 25 is transmitted to the opening/closing mechanism 51 through the transmission mechanism. The third driver 25 drives the opening/closing mechanism 51, thereby changing the opening/closing state of the grasper 50.

The robot arm 2 also includes a sensor 26 for detecting the posture of the robot arm 2. The sensor 26 may detect, for example, an angle at which each articulation 22 rotates, or a state (e.g., compressed air pressure) of the first driver 23 driving each articulation 22. The sensor 26 also outputs a sensor signal indicating the detection result to the controller 3.

### [4. Operation Unit]

The operation unit 4 is configured to receive operations of the robot arm 2 and the surgical tool 5 from the user and includes two operation devices 40 corresponding to the two robot arms 2, a display 43, and an input section 44 (see FIG. 2). Each operation device 40 includes a position operation section 41 and a grasp operation section 42.

The position operation section 41 receives an operation for adjusting the position and orientation of the grasper 50 of the surgical tool 5, which is mounted on the robot arm 2 corresponding to the operation device 40 provided with the position operation section 41. That is, in response to the operation applied to the position operation section 41, the posture of the robot arm 2 is changed to thereby adjust the position and orientation of the surgical tool 5 and the wrist 52 of the surgical tool 5 is actuated to thereby adjust the orientation of the grasper 50.

The grasp operation section 42 receives an operation to cause the grasper 50 of the surgical tool 5, which is mounted on the robot arm 2 corresponding to the operation device 40 provided with the grasp operation section 42, to perform the opening/closing movement.

Then, the operation device 40 outputs, to the controller 3, an operation signal indicating the contents of the operations received by the position operation section 41 and the grasp operation section 42.

As described above, the surgical robot 1 is configured to perform force feedback. Thus, the position operation section 41 and the grasp operation section 42 provide the user with a reaction force corresponding to a force received by the surgical tool 5 from the subject or the like when the surgical tool 5 is actuated in accordance with the user's operation.

Specifically, the position operation section 41 includes a movable part 41A and a driver 41B, and the grasp operation section 42 includes a movable part 42A and a driver 42B. The drivers 41B, 42B drive the movable parts 41A, 42A by, for example, a pneumatic actuator, a motor, or the like. The driver 41B of the position operation section 41 displaces the movable part 41A in accordance with the control signal input from the controller 3, thereby providing the reaction force to the user operating the position operation section 41. Similarly, the driver 42B of the grasp operation section 42 displaces the movable part 42A in accordance with the control signal input from the controller 3, thereby providing the reaction force to the user operating the grasp operation section 42.

The display 43 is configured as a liquid crystal display, for example, and is actuated in accordance with a control signal from the controller 3 to display, for example, a setting screen, an endoscope image of the subject, the states of the robot arm 2 and the surgical tool 5, and information necessary for operating the surgical robot 1. The endoscope image may be, for example, an image of the patient's body cavity obtained from an endoscope inserted into the patient's body cavity through another trocar punctured in the patient's abdomen or the like. This endoscope may be held by another robot arm or an endoscope holding device that is not shown.

The input section 44 is a device for receiving an operation from the user and the like, and includes, for example, a keyboard, a mouse, a touch panel, a foot switch, or the like. The input section 44 outputs an operation signal indicating the content of the received operation to the controller 3.

### [5. Controller]

### [(1) Overall Configuration]

The controller 3 is configured to control each robot arm 2, each surgical tool 5, and the operation unit 4 (see FIG. 2). The controller 3 is configured as a computer system equipped with a processor, ROM, RAM, a bus, an input/output section, and the like. The functions of the controller 3 are realized by running a processor according to a program stored in the ROM or the RAM. Some or all of the functions realized by the processor may be realized by hardware, such as at least one IC and the like.

The controller 3 includes a master controller 30, a slave controller 31, a gain adjustor 32, and a storage device 33.

The storage device 33 is a non-volatile storage medium, such as a hard disk and a flash memory, and stores a program necessary for processing by the controller 3 and information about the operation of the surgical robot 1.

Here, the functions of the master controller 30 and the slave controller 31 are realized by running the processor. The master controller 30 is configured to control the operation unit 4, and the slave controller 31 is configured to control each robot arm 2 and the surgical tool 5 mounted on each robot arm 2. As described in detail below, the master controller 30 and the slave controller 31 perform a master-slave control to thereby calculate a master driving force τm (i.e., master control value) and a slave driving force τs (i.e., a slave control value).

The master driving force τm represents the driving forces of the drivers 41B, 42B of the position operation section 41 and the grasp operation section 42 in each operation device 40 of the operation unit 4. The slave driving force τs represents the driving forces of the first to third drivers 23-25 in each robot arm 2.

The master controller 30 outputs control signals to the drivers 41B, 42B of the position operation section 41 and the grasp operation section 42, and controls the drivers 41B, 42B to respectively actuate the movable parts 41A, 42A with the driving forces τm, thereby generating the reaction forces.

The master controller 30 also detects the operations performed by the user on the position operation section 41 and the grasp operation section 42 based on operation signals from the operation unit 4. The master controller 30 then determines a target state (i.e., Xm) regarding the current position and orientation and the opening/closing movement of the grasper 50 based on the detected operations. The details of the target state and Xm are described below.

Here, the slave controller 31 outputs control signals to the first to third drivers 23-25 of the robot arm 2. This causes the slave controller 31 to control the first and the second drivers 23, 24 to actuate the plurality of articulations 22 and the wrist 52 with the driving forces τs. The slave controller 31 controls the third driver 25 to actuate the opening/closing mechanism 51 with the driving force τs.

The slave controller 31 also determines a current state of the opening/closing movement of the grasper 50 as a surgical tool state (i.e., Xs) based on a sensor signal from the sensor 50A provided to the grasper 50 of the surgical tool 5. The slave controller 31 also determines the current position and orientation of the grasper 50 as the surgical tool state (i.e., Xs) based on a sensor signal from the sensor 52A provided to the wrist 52 of the surgical tool 5 and a sensor signal from the sensor 26 provided to the robot arm 2. The details of the surgical tool state and Xs are described below.

### [(2) Asymmetric Bilateral Control]

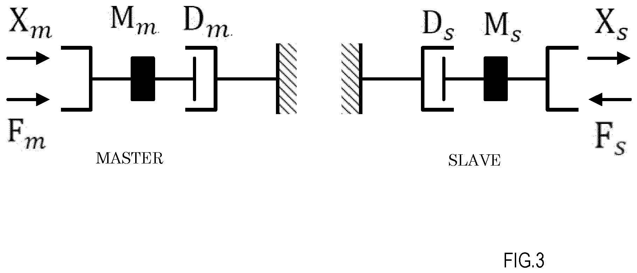

The master and the slave of this embodiment can be modeled as a one-degree-of-freedom system shown in FIG. 3. Here, the dynamics of the master and the slave are represented by the following equations.
[Mathematical 1] ${τ}_{m} + {F}_{m} = {M}_{m} {{X}^{¨}}_{m} + {D}_{m} {\dot{X}}_{m}$ (1)
[Mathematical 2] ${τ}_{s} - {F}_{s} = {M}_{s} {{X}^{¨}}_{s} + {D}_{s} {\dot{X}}_{s}$ (2)

Here, in the control of the position and orientation of the grasper 50, the position operation section 41 used for operations corresponds to the master, and the grasper 50 corresponds to the slave. In the control of the opening/closing movement of the grasper 50, the grasp operation section 42 used for operations corresponds to the master, and the grasper 50 corresponds to the slave.

Xm represents a state to be achieved by the slave (i.e., a target state of the slave) determined based on a current state of the master determined by the operation from the user. More specifically, the target state corresponds to target values of the position and orientation of the grasper 50 and a target value of the opening/closing state. Mm represents the mass of the master, Dm represents a viscous friction coefficient of a damper on the master side, and Fm represents an external force applied to the master by the user's operation. As described above, τm represents the driving force when the driver actuates the movable part to generate the reaction force in the position operation section 41 and the grasp operation section 42.

Xs represents a current state of the slave (i.e., a surgical tool state). More specifically, the surgical tool state corresponds to values indicating the current position and orientation of the grasper 50 and a value indicating the current opening/closing state. Ms represents the mass of the slave, Ds represents a viscous friction coefficient of a damper on the slave side, and Fs represents an external force applied to the slave by the subject during actuation. As described above, τs represents the driving forces when the first and second drivers 23, 24 actuate the articulation 22 and the wrist 52 to cause the position and orientation of the grasper 50 to achieve the target state. In addition, τs also represents the driving force when the third driver 25 actuates the opening/closing mechanism 51 to cause the opening/closing state of the grasper 50 to achieve the target state.

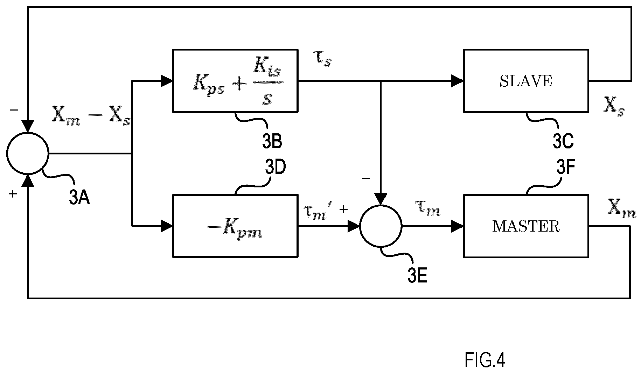

The master controller 30 and the slave controller 31 perform the asymmetric bilateral control as shown in the block diagram of FIG. 4. In this control, Xs is subtracted from Xm at a summing point 3A to calculate a difference, and the difference is input to a transfer element 3B. The transfer element 3B calculates a slave driving force τs by a proportional control and an integral control based on the difference and outputs the driving force τs to both a transfer element 3C representing the slave and a summing point 3E. The transfer element 3C outputs Xs to the summing point 3A.

The difference between Xm and Xs calculated at the summing point 3A is also input to a transfer element 3D that performs the proportional control, and the transfer element 3D calculates τm' by the proportional control based on the difference and outputs τm' to the summing point 3E. At the summing point 3E, the slave driving force τs is subtracted from τm' to calculate a master driving force τm, and the driving force τm is output to a transfer element 3F representing the master. The transfer element 3F outputs Xm to the summing point 3A.

The transfer element 3B corresponds to a process in the slave controller 31, and performs the proportional control with a gain Kps and the integral control with a gain Kis. The transfer element 3D and the summing point 3E correspond to processes in the master controller 30, and the transfer element 3D performs the proportional control with a gain -Kpm.

That is, the master controller 30 calculates the master driving force τm based on Xm, Xs, and the slave driving force τs obtained from the slave controller 31. The absolute value of -Kpm (hereinafter, | Kpm |) is greater than the absolute value of Kps (hereinafter, | Kps |). More specifically, as an example, | Kpm | may be two times or more and three times or less than | Kps |. However, | Kpm | and | Kps | are not limited thereto, and may be determined as appropriate. For example, | Kpm | may be smaller than | Kps |.

A driving force τm after scaling may be input into the transfer element 3F representing the master. The "scaling" means multiplying the master driving force τm by a coefficient of a specified value greater than 1. This scaling allows a reaction force greater than the force received by the slave to be applied to the user, and allows the user to appropriately perform a treatment on a minute part of the subject's body and a treatment that requires precision.

The block diagram of FIG. 3 is represented by the following equations (3) and (4).
[Mathematical 3] ${τ}_{m} = \left({K}_{pm}+{K}_{ps}\right) \left({X}_{s}-{X}_{m}\right) + {K}_{is} \int \left({X}_{s}-{X}_{m}\right) dt$ (3)
[Mathematical 4] ${τ}_{s} = {K}_{ps} \left({X}_{m}-{X}_{s}\right) + {K}_{is} \int \left({X}_{m}-{X}_{s}\right) dt$ (4)

Here, by substituting the equations (3) and (4) into the equations (1) and (2) and by performing a Laplace transformation, the following equation (5) can be obtained.
[Mathematical 5] $\begin{matrix}{F}_{m} \left(s\right) = {M}_{m} {X}_{m} {s}^{2} + {D}_{m} {X}_{m} s - {τ}_{m} \\ \\ = {M}_{m} {X}_{m} {s}^{2} + {D}_{m} {X}_{m} s + \left({K}_{pm}+{K}_{ps}+\frac{{K}_{is}}{s}\right) \left({X}_{m}-{X}_{s}\right) \\ = {M}_{m} {X}_{m} {s}^{2} + {D}_{m} {X}_{m} s + G \left(s\right) \left({M}_{s}{{X}^{¨}}_{s}+{D}_{s}{\dot{X}}_{s}+{F}_{s}\right)\end{matrix}$ (5)

Note that G(s) satisfies the following equation (6).
[Mathematical 6] $G \left(s\right) = \frac{\left({K}_{pm}+{K}_{ps}\right) + {K}_{is}}{{K}_{pm} s + {K}_{is}}$ (6)

Here, if T and a satisfy equations (7) and (8), an equation (9) holds.
[Mathematical 7] $T = \frac{{K}_{ps}}{{K}_{is}}$ (7)
[Mathematical 8] $a = \frac{{K}_{pm} + {K}_{ps}}{{K}_{ps}}$ (8)
[Mathematical 9] $G \left(s\right) = \frac{1 + saT}{1 + sT}$ (9)

That is, the equation (9) shows that the asymmetric bilateral control has phase-lead compensation characteristics. Thus, by setting the gain | Kpm | of the proportional control of the master greater than the gain | Kps | of the proportional control of the slave, a gain of G(s) is "a" in a high-frequency range and "1" in a low-frequency range. That is, when the external force Fs applied to the slave changes significantly, such as at the moment when the slave comes in contact with an object, a force multiplied by "a" is fed back to the master, and a force multiplied by "1" is fed back to the master in a steady state. Therefore, the asymmetric bilateral control promotes both highly accurate slave tracking performance through the integral control and highly sensitive force feedback.

### [(3) Variation of Master Slave Control]

As shown in equations (10) and (11), the acceleration and the velocity are used in each of the equations (3) and (4), whereby an inertial force and a viscous force may be compensated.
[Mathematical 10] ${τ}_{m} = \left({K}_{pm}+{K}_{ps}\right) \left({X}_{s}-{X}_{m}\right) + {K}_{is} \int \left({X}_{s}-{X}_{m}\right) dt + \left({M}_{m}{{X}^{¨}}_{m}+{D}_{m}{\dot{X}}_{m}\right)$ (10)
[Mathematical 11] ${τ}_{s} = {K}_{ps} \left({X}_{m}-{X}_{s}\right) + {K}_{is} \int \left({X}_{m}-{X}_{s}\right) dt + \left({M}_{s}{{X}^{¨}}_{s}+{D}_{s}{\dot{X}}_{s}\right)$ (11)

From the equations (10) and (11), an equation (12) is obtained.
[Mathematical 12] ${F}_{m} \left(s\right) = G \left(s\right) {F}_{s}$ (12)

From the equation (12), it can be seen that the negative effects of the dynamics of the master and the slave can be reduced by adding compensation as in the equations (10) and (11).

Moreover, although the slave driving force τs is calculated using the proportional control and the integral control in the slave controller 31, the slave driving force τs may be calculated using a disturbance observer in place of the integral control.

In the asymmetric bilateral control, the master controller 30 calculates the master driving force τm by subtracting the slave driving force τs from τm' output from the transfer element 3D. However, the calculation method of τm is not limited thereto, and can be determined as appropriate. Specifically, for example, a specified calculation may be performed based on the slave driving force τs to calculate τs', and τs' may be subtracted from τm' to thereby calculate τm. The specified calculation may involve, for example, multiplying τs by a specified coefficient or adding a specified coefficient to τs. For example, τm may also be calculated by multiplying τm' by τs (or τs'), dividing τm' by τs (or τs'), or adding τs (or τs') to τm'.

In the transfer element 3B, τs may be calculated without using the integral control and the disturbance observer. In the transfer elements 3B and 3D, a derivative control may be further performed to calculate τs and τm', respectively.

### [(4) Gain Adjustment]

The function of the gain adjustor 32 is realized by running the processor of the controller 3 (see FIG. 2). The gain adjustor 32 adjusts the gains of the integral control and the proportional control in the asymmetric bilateral control in accordance with the operation received from the user or the like through the input section 44.

That is, the storage device 33 stores the gains Kpm of the proportional control in the master controller 30 and the gains Kps of the proportional control and the gains Kis of the integral control in the slave controller 31 for each robot arm 2. For example, when the surgical robot 1 is started up or the like, the controller 3 reads the gains Kpm, Kps, and Kis corresponding to each robot arm 2 from the storage device 33, and performs the above-described bilateral control using the read gains Kpm, Kps and Kis.

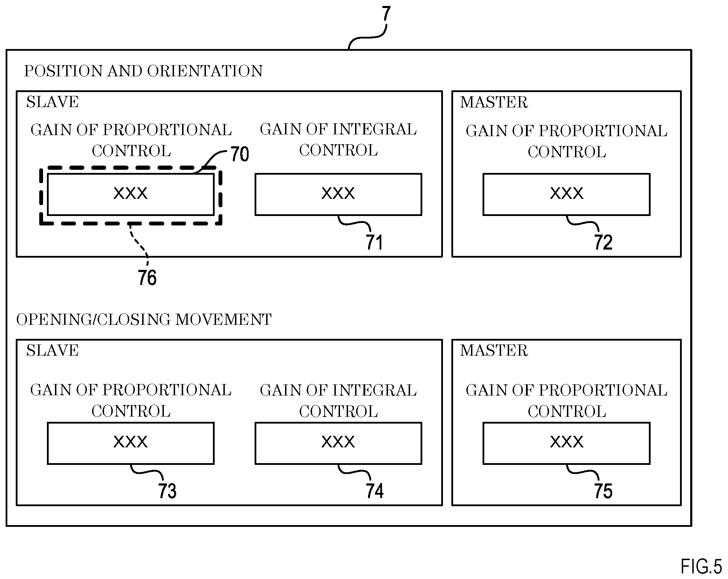

When receiving an operation signal indicating an instruction to adjust the gain(s) from the input section 44, the gain adjustor 32 outputs a control signal to the display 43 to show a gain setting screen 7 on the display 43 (see FIG. 5). The gain setting screen 7 is provided for each robot arm 2, and is used to adjust the gain(s) in the bilateral control of the corresponding robot arm 2. In accordance with an operation signal input from the input section 44, the gain adjustor 32 outputs a control signal to the display 43 to switch the gain setting screen 7 shown on the display 43 to a gain setting screen 7 corresponding to another robot arm 2.

The gain setting screen 7 includes display areas 70, 71 for the gains of the proportional and integral controls of the slave, and a display area 72 for the gain of the proportional control of the master, related to the control of the position and orientation of the grasper 50. The gain setting screen 7 also includes display areas 73, 74 for the gains of the proportional and integral controls of the slave, and a display area 75 for the gain of the proportional control of the master, related to the control of the opening/closing state of the grasper 50. The gain adjustor 32 outputs a control signal to the display 43 to cause these display areas 70-75 to show current values of the gains in the corresponding proportional control or integral control.

The gain setting screen 7 also shows a cursor 76 that is superimposed on any one of the display areas 70-75. In accordance with an operation signal input from the input section 44, the gain adjustor 32 selects one of the display areas 70-75 and outputs a control signal to the display 43 to cause it to show the cursor 76 superimposed on the selected display area. In accordance with an operation signal input from the input section 44, the gain adjustor 32 accepts a change to the gain value displayed in the selected display area, and outputs a control signal to the display 43 to cause it to show the changed gain value in the display area.

When an operation signal indicating the confirmation of the gain values is input from the input section 44, the gain adjustor 32 updates the gain values of the proportional control or the integral control corresponding to the display areas 70-75 to the gain values displayed in the display areas. Thereafter, the updated gain values are used in the bilateral control. The gain adjustor 32 stores each updated gain value in the storage device 33.

### [6. Effects]

(1) In the conventional bilateral control, a symmetric or force feedback method using the proportional control and the derivative control has been widely used. In order to improve the accuracy of the control, when forceps are used as a surgical tool, it is necessary to minimize an error as much as possible even if a large external force (e.g., external force of about 5-20 N) is applied to the tip of the forceps. To this end, it is necessary to increase the gain of the proportional control in the slave control; however, as described above, there is a limit to increasing the gain of the proportional control. Thus, it is necessary to control the slave by an integral-based method, such as the integral control or the disturbance observer; however, introducing such a method may delay the force feedback.
   In contrast, in the asymmetric bilateral control in this embodiment, the slave controller 31 calculates τs by the proportional control and the integral control. Thus, disturbances can be well suppressed in the slave controller 31, and the accuracy of τs improves. The master controller 30 calculates τm based on τs obtained from the slave controller 31, and uses τm to control the reaction force generated in the position operation section 41 and the grasp operation section 42, which serve as the master. Therefore, the sensitivity of the force feedback improves. That is, in this embodiment, even though the slave is controlled by the integral-based method, the delay in the force feedback can be reduced.
(2) The gain | Kpm | of the proportional control of the master is greater than the gain | Kps | of the proportional control of the slave. Therefore, the sensitivity of the force feedback improves.
(3) The gain adjustor 32 adjusts each gain in the bilateral control corresponding to each robot arm in accordance with the operation received through the input section 44. This allows the adjustment of the sensitivity of the force feedback and the customization for better control of the surgical tool.

### [7. Other Embodiments]

Two or more functions of one element of the aforementioned embodiment may be achieved by two or more elements, and one function of one element may be achieved by two or more elements. Furthermore, two or more functions of two or more elements may be achieved by one element, and one function achieved by two or more elements may be achieved by one element. A part of the configurations of the aforementioned embodiment may be omitted. Furthermore, at least a part of the configurations of the aforementioned embodiment may be added to or replaced by another configuration of the aforementioned embodiment.

### [8. Correspondence between Terms]

The position operation section 41 and the grasp operation section 42 of the operation device 40 correspond to examples of the master. The first to third drivers 23-25 of the robot arm 2 correspond to examples of the slave, and the sensor 26 of the robot arm 2 and the sensors 50A, 52A of the surgical tool correspond to examples of the slave detector.

## Claims

1. A controller (3) for a surgical robot (1), the controller (3) comprising:
a master controller (30) configured to control a master (41, 42) based on a master control value, the master (41, 42) being configured to receive an operation from a user and to apply a reaction force to the user performing the operation, the master controller (30) being configured to cause the reaction force to be generated, the master control value being for controlling the reaction force; and
a slave controller (31) configured to control a slave (23~25) based on a target state, the slave (23~25) being configured to actuate a surgical tool (5), the target state being a state of the surgical tool (5) based on the operation received by the master (41, 42), the slave controller (31) being configured to cause the surgical tool (5) to be actuated,
wherein the slave controller (31) is configured to calculate a slave control value based on a surgical tool state and the target state, the slave control value being for controlling the surgical tool (5), the surgical tool state being a state of the surgical tool (5) detected by a slave detector (50A, 52A),
the slave controller (31) is configured to provide the slave control value to the master controller (30), and to control the slave (23~25) based on the slave control value provided to the master controller (30), and **characterized in that**
wherein the master controller (30) is configured to calculate the master control value based on the surgical tool state, the target state, and the slave control value obtained from the slave controller (31) .

2. The surgical robot (1) including the controller (3) according to claim 1, the surgical robot comprising:
the master (41, 42);
the slave (23~25);
the slave detector (50A, 52A); and
the controller (3).

3. The surgical robot (1) according to claim 2,
wherein the slave controller (31) is configured to calculate the slave control value by performing an integration based on the surgical tool state and the target state.

4. The surgical robot (1) according to claim 2 or 3,
wherein the slave controller (31) is configured to calculate the slave control value by a proportional control based on a difference between the surgical tool state and the target state,
wherein the master controller (30) is configured to calculate the master control value by a proportional control based on the difference between the surgical tool state and the target state, and
wherein a gain of the proportional control in the master controller (30) is greater than a gain of the proportional control in the slave controller (31).

5. The surgical robot (1) according to any one of claims 2 to 4,
wherein the master controller (30) is configured to calculate the master control value by a proportional control based on a difference between the surgical tool state and the target state, and
wherein the surgical robot further comprises
a display (43) configured to display a gain of the proportional control and
an input section (44) configured to receive an operation to change the gain of the proportional control.

6. The surgical robot (1) according to any one of claims 2 to 5,
wherein the slave controller (31) is configured to calculate the slave control value by a proportional control based on a difference between the surgical tool state and the target state, and
wherein the surgical robot (1) further comprises
a display (43) configured to display a gain of the proportional control and
an input section (44) configured to receive an operation to change the gain of the proportional control.

7. The surgical robot (1) according to claim 6,
wherein the slave controller (31) is configured to calculate the slave control value by further performing an integral control based on the difference between the surgical tool state and the target state,
wherein the display (43) is configured to further display a gain of the integral control, and
wherein the input section (44) is configured to further receive an operation to change the gain of the integral control.

## Patentansprüche

1. Controller (3) für einen Chirurgieroboter (1), wobei der Controller (3) umfasst:
einen Master-Controller (30), der konfiguriert ist, einen Master (41, 42) basierend auf einem Master-Control-Wert zu steuern, wobei der Master (41, 42) konfiguriert ist, eine Operation von einem Benutzer zu empfangen und eine Reaktionskraft auf den die Operation ausführenden Benutzer anzulegen, wobei der Master-Controller (30) konfiguriert ist, zu veranlassen, dass die erzeugte Reaktionskraft erzeugt wird, wobei der Master-Control-Wert zur Steuerung der Reaktionskraft dient; und
einen Slave-Controller (31) konfiguriert ist, einen Slave (23~25) basierend auf einem Ziel-Zustand zu steuern, wobei der Slave (23~25) konfiguriert ist, ein Chirurgieinstrument zu aktivieren (5), wobei der Ziel-Zustand der Zustand des Chirurgieinstruments (5) basierend auf der vom Master empfangenen Operation (41, 42) ist, wobei der Slave-Controller (31) konfiguriert ist, zu veranlassen, dass das Chirurgieinstrument (5) betätigt wird,
wobei der Slave-Controller (31) konfiguriert ist, einen Slave-Control-Wert basierend auf einem Chirurgieinstrument-Zustand und dem Ziel-Zustand zu berechnen, wobei der Slave-Control-Wert zur Steuerung des Chirurgieinstruments (5) dient, wobei der Chirurgieinstrument-Zustand ein Zustand des Chirurgieinstruments (5) ist, der von einem Slave-Detektor (50A, 52A) erkannt wird,
der Slave-Controller (31) konfiguriert ist, dass er den Slave-Control-Wert dem Master-Controller (30) bereitstellt und den Slave (23~25) basierend auf dem von dem Master Controller (30) bereitgestellten Slave-Control-Wert steuert, und **dadurch gekennzeichnet dass**
der Master-Controller (30) konfiguriert ist, den Master-Control-Wert basierend auf dem Chirurgieinstrument-Zustand, dem Ziel-Zustand und dem vom Slave-Controller (31) erhaltenen Slave-Control-Wert zu berechnen.

2. Chirurgieroboter (1), der den Controller (3) gemäß Anspruch 1 einschließt, wobei der Chirurgieroboter umfasst:
den Master (41, 42);
den Slave (23~25);
den Slave-Detektor (50A, 52A); und
den Controller (3).

3. Chirurgieroboter (1) gemäß Anspruch 2,
wobei der Slave-Controller (31) konfiguriert ist, den Slave-Control-Wert durch Ausführen einer Integration basierend auf dem Chirurgieinstrument-Zustand und dem Ziel-Zustand zu berechnen.

4. Chirurgieroboter (1) gemäß Anspruch 2 oder 3,
wobei der Slave-Controller (31) konfiguriert ist, den Slave-Control-Wert durch eine Proportionalsteuerung basierend auf einer Differenz zwischen dem Chirurgieinstrument-Zustand und dem Ziel-Zustand zu berechnen,
wobei der Master-Controller (30) konfiguriert ist, den Master-Control-Wert durch eine Proportionalsteuerung basierend auf der Differenz zwischen dem Chirurgieinstrument-Zustand und dem Ziel-Zustand zu berechnen, und
wobei eine Verstärkung der Proportionalsteuerung im Master-Controller (30) größer ist als eine Verstärkung der Proportionalsteuerung im Slaven-Controller (31).

5. Chirurgieroboter (1) gemäß einem der Ansprüche 2 bis 4,
wobei der Master-Controller (30) konfiguriert ist, den Master-Control-Wert durch eine Proportionalsteuerung basierend auf einer Differenz zwischen dem Chirurgieinstrument-Zustand und dem Ziel-Zustand zu berechnen, und
wobei der Chirurgieroboter weiterhin umfasst
eine Anzeige (43), die konfiguriert ist, eine Verstärkung der Proportionalsteuerung anzuzeigen, und
eine Eingangssektion (44), die konfiguriert ist, eine Operation zur Änderung der Verstärkung der Proportionalsteuerung zu empfangen.

6. Chirurgieroboter (1) gemäß einem der Ansprüche 2 bis 5,
wobei der Slave-Controller (31) konfiguriert ist, den Slave-Control-Wert durch eine Proportionalsteuerung basierend auf einer Differenz zwischen dem Chirurgieinstrument-Zustand und dem Ziel-Zustand zu berechnen, und
wobei der Chirurgieroboter (1) weiterhin umfasst
eine Anzeige (43), die konfiguriert ist, eine Verstärkung der Proportionalsteuerung anzuzeigen, und
eine Eingangssektion (44), die konfiguriert ist, eine Operation zur Änderung der Verstärkung der Proportionalsteuerung zu empfangen.

7. Chirurgieroboter (1) gemäß Anspruch 6,
wobei der Slave-Controller (31) konfiguriert ist, den Slave-Control-Wert durch weiterhin Ausführen einer Integralsteuerung basierend auf der Differenz zwischen dem Chirurgieinstrument-Zustand und dem Ziel-Zustand zu berechnen,
wobei die Anzeige (43) konfiguriert ist, eine Verstärkung der Integralsteuerung weiterhin anzuzeigen, und
wobei die Eingangssektion (44) konfiguriert ist, eine Operation zur Änderung der Verstärkung der Integralsteuerung weiterhin zu empfangen.

## Revendications

1. Un contrôleur (3) pour un robot chirurgical (1), le contrôleur (3) comprenant :
un contrôleur maître (30) configuré pour contrôler un maître (41, 42) basé sur une valeur de contrôle maître, le maître (41, 42) étant configuré pour recevoir une opération d'un utilisateur et appliquer une force de réaction à l'utilisateur effectuant l'opération, le contrôleur maître (30) étant configuré pour générer la force de réaction, la valeur de contrôle maître étant pour le contrôle de la force de réaction ; et
un contrôleur esclave (31) configuré pour contrôler un esclave (23~25) basé sur un état cible, l'esclave (23~25) étant configuré pour actionner un outil chirurgical (5), l'état cible étant un état de l'outil chirurgical (5) basé sur l'opération reçue par le maître (41, 42), le contrôleur esclave (31) étant configuré pour provoquer l'outil chirurgical (5) à être actionné,
dans lequel le contrôleur esclave (31) est configuré pour calculer une valeur de contrôle esclave basée sur l'état d'un outil chirurgical et l'état cible, la valeur de contrôle esclave étant pour le contrôle de l'outil chirurgical (5), l'état de l'outil chirurgical étant un état de l'outil chirurgical (5) détecté par un détecteur d'esclave (50A, 52A),
le contrôleur esclave (31) est configuré pour fournir la valeur de contrôle esclave au contrôleur maître (30), et pour contrôler l'esclave (23~25) en fonction de la valeur de contrôle esclave fournie au contrôleur maître (30), et **caractérisé en ce que**
le contrôleur maître (30) est configuré pour calculer la valeur de contrôle maître basée sur l'état de l'outil chirurgical, de l'état cible et de la valeur de contrôle esclave obtenue auprès du contrôleur esclave (31 ).

2. Le robot chirurgical (1) incluant le contrôleur (3) selon l'affirmation 1, le robot chirurgical comprenant :
le maître (41, 42) ;
l'esclave (23~25) ;
le détecteur d'esclave (50A, 52A) ; et
le contrôleur (3).

3. Le robot chirurgical (1) selon la revendication 2,
dans lequel le contrôleur esclave (31) est configuré pour calculer la valeur de contrôle esclave en effectuant une intégration basée sur l'état de l'outil chirurgical et l'état cible.

4. Le robot chirurgical (1) selon la revendication 2 ou 3,
dans lequel le contrôleur esclave (31) est configuré pour calculer la valeur de contrôle esclave par un contrôle proportionnel basé sur une différence entre l'état de l'outil chirurgical et l'état cible,
dans lequel le contrôleur maître (30) est configuré pour calculer la valeur de contrôle maître par un contrôle proportionnel basé sur la différence entre l'état de l'outil chirurgical et l'état cible, et
dans lequel un gain du contrôle proportionnel dans le contrôleur maître (30) est supérieur au gain du contrôle proportionnel dans le contrôleur esclave (31).

5. Le robot chirurgical (1) selon l'une des revendications2 à 4,
dans lequel le contrôleur maître (30) est configuré pour calculer la valeur de contrôle maître par un contrôle proportionnel basé sur une différence entre l'état de l'outil chirurgical et l'état cible, et
dans lequel le robot chirurgical comprend en outre
un affichage (43) configuré pour afficher un gain du contrôle proportionnel et
une section d'entrée (44) configurée pour recevoir une opération modifiant le gain du contrôle proportionnel.

6. Le robot chirurgical (1) selon l'une des revendications2 à 5,
dans lequel le contrôleur esclave (31) est configuré pour calculer la valeur de contrôle esclave par un contrôle proportionnel basé sur une différence entre l'état de l'outil chirurgical et l'état cible, et
dans lequel le robot chirurgical (1) comprend en outre
un affichage (43) configuré pour afficher un gain du contrôle proportionnel et
une section d'entrée (44) configurée pour recevoir une opération modifiant le gain du contrôle proportionnel.

7. Le robot chirurgical (1) selon la revendication 6,
dans lequel le contrôleur esclave (31) est configuré pour calculer la valeur de contrôle esclave en effectuant un contrôle intégral basé sur la différence entre l'état de l'outil chirurgical et l'état cible,
dans lequel l'affichage (43) est configuré pour afficher en outre un gain supplémentaire du contrôle intégral, et
dans lequel la section d'entrée (44) est configurée pour recevoir en outre une opération modifiant le gain du contrôle intégral.
